(19) Europäisches Patentamt
European Patent Office
Office européen des brevets

(11) Veröffentlichungsnummer: **0 156 773 B1**

(12) # EUROPÄISCHE PATENTSCHRIFT

(45) Veröffentlichungstag der Patentschrift: **06.09.89**

(51) Int. Cl.⁴: **C 07 C 131/00**, C 07 C 147/02, C 07 C 147/06, A 01 N 35/10, C 07 C 149/20

(21) Anmeldenummer: **85810131.4**

(22) Anmeldetag: **25.03.85**

(54) **Cyclohexenon-carbonsäurederivate mit herbizider und das Pflanzenwachstum regulierender Wirkung.**

(30) Priorität: **30.03.84 CH 1613/84**

(43) Veröffentlichungstag der Anmeldung: **02.10.85 Patentblatt 85/40**

(45) Bekanntmachung des Hinweises auf die Patenterteilung: **06.09.89 Patentblatt 89/36**

(84) Benannte Vertragsstaaten: **BE CH DE FR GB IT LI NL**

(56) Entgegenhaltungen:
**EP-A- 0 080 301**
**DE-A- 2 461 027**
**FR-A- 2 494 688**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Brunner, Hans-Georg, Dr., Wannenstrasse 14, CH-4415 Lausen (CH)**

**EP 0 156 773 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft neue Cyclohexandion-carbonsäurederivate mit herbizider und das Pflanzenwachstum regulierender Wirkung, Mittel welche diese Cyclohexandioncarbonsäurederivate enthalten sowie die Verwendung dieser Derivate zur selektiven und totalen Unkrautbekämpfung und zur Regulierung des Pflanzenwachstums.

Die neuen Cyclohexandioncarbonsäurederivate entsprechen der Formel I

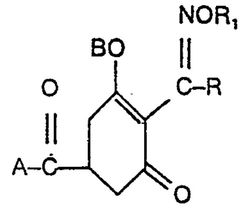

(I),

worin

A einen Rest $-OR_2$ oder $-NR_3R_4$,

R $C_1$–$C_6$ Alkyl oder $C_3$–$C_6$ Cycloalkyl, unsubstituiert oder substituiert durch Halogen, $C_1$–$C_4$ Alkoxy oder $C_1$–$C_4$ Alkylthio,

$R_1$ $C_1$–$C_6$ Alkyl, $C_1$–$C_6$-Halogenalkyl, $C_3$–$C_6$ Alkenyl, $C_3$–$C_6$-Halogenalkenyl oder $C_3$–$C_6$ Alkinyl,

$R_2$ und $R_3$ unabhängig voneinander je Wasserstoff; $C_1$–$C_6$ Alkyl, $C_1$–$C_6$ Halogenalky, $C_2$–$C_{10}$ Alkoxyalkyl, $C_2$–$C_{10}$ Aklythioalkyl; $C_3$–$C_6$ Alkenyl, unsubstituiert oder substituiert durch Halogen, $C_1$–$C_4$ Alkoxy oder $C_1$–$C_4$ Alkylthio; $C_3$–$C_6$ Alkinyl; Phenyl oder $C_1$–$C_6$ Aralkyl, wobei der Phenylkern unsubstituiert oder durch Halogen, $C_1$–$C_4$ Alkyl, $C_1$–$C_4$ Alkoxy, $C_1$–$C_4$ Halogenalkyl, Nitro oder Cyan substituiert ist,

$R_4$ $C_1$–$C_4$-Alkoxy oder dasselbe wie $R_2$,

$R_3$ und $R_4$ zusammen mit dem Stickstoffatom, an das sie gebunden sind, bilden auch einen 5–6 gliedrigen Heterocyclus, der im Ring noch ein Sauerstoff- oder Schwefelatom enthalten kann,

B einen Rest $-COR_5$, $-SO_2R_5$ oder $R_6$,

$R_5$ $C_1$–$C_6$Alkyl, $C_3$–$C_6$Alkenyl, $C_3$–$C_6$Alkinyl, sowie Phenyl oder Benzyl, welches unsubstituiert oder durch Halogen, $C_1$–$C_4$Alkyl, $C_1$–$C_4$Halogenalkyl, $C_1$–$C_4$Alkoxy, $C_1$–$C_4$Halogenalkoxy, Nitro oder Cyano substituiert sein kann,

$R_6$ $C_1$–$C_6$Alkyl, $C_3$–$C_6$Alkenyl, $C_3$–$C_6$Alkinyl oder auch Benzyl, welches unsubstituiert oder wie oben substituiert sein kann.

In diesen Definitionen umfassen die Alkylreste sowohl geradkettige wie verzweigte, z.B. Methyl, Äthyl, Propyl, Isopropyl, Butyl, Isobutyl, sec. Butyl, tert.Butyl sowie alle stereoisomeren Formen der höheren Homologen. Ebenso werden unter Alkenyl und Alkinyl geradkettige wie verzweigte Reste verstanden, z.B. Vinyl, Allyl, Methallyl, Butenyl, Methyl-, und Dimethylbutenyl, Äthinyl, Propinyl, Butinyl, Methylbutinyl, Dimethylbutinyl.

Unter Halogen ist Fluor-, Chlor-, Brom- oder Jodatome zu verstehen.

Unter den von $R_3$ und $R_4$ zusammen mit dem Stickstoffatom gebildeten 5–6 gliedrigen Heterocyclen, welche im Ring noch ein Sauerstoff- oder Schwefelatom enthalten können, sind Pyrrol, Pyrrolidin, Piperidin, Morpholin oder auch Thiomorpholin-Reste zu verstehen. Solche Ringe können durch Methylgruppen substituiert sein.

Die Cyclohexenoncarbonsäurederivate der Formel I zeichnen sich durch gute herbizide und den Pflanzenwuchs regulierende Wirkung aus. Unter den Verbindungen, die durch ihre Wirkung besonders aufgefallen sind, befinden sich folgende Gruppen:

- Cyclohexanderivate der Formel Ia
- die Verbindungen der Formel I, in denen A einen Aminorest $-NR_3R_4$ und B einen Acylrest $-COR_5$ bedeutet, besonders
  3-Acetoxy-4-(1-äthoximino-butyl)-5-oxo-cyclohex-3-en-carbonsäuredimethylamid und
  3-Benzyloxy-4-(1-äthoximino-butyl)-5-oxo-cyclohex-3-en-carbonsäuredimethylamid;
- die Verbindungen der Formel I, in denen A einen Aminorest $-NR_3R_4$ und B einen Rest $-R_6$ bedeutet, besonders
  3-Methoxy-4-(1-Äthoximino-butyl)-5-oxo-cyclohex-3-en-carbonsäuredimethylamid;
- die Verbindungen der Formel I, in denen A einen Aminorest $-NR_3R_4$ und B einen Sulfonylrest $-SR_2R_5$ bedeutet;
- die Verbindungen der Formel I, in denen A einen Alkanolrest $-OR_2$ und B einen Acylrest $-COR_5$ bedeutet;
- die Verbindungen der Formel I, in denen A einen Alkanolrest $-OR_2$ und B einen Sulfonylrest $-SO_2R_5$ bedeutet;
- die Verbindungen der Formel I, in denen A einen Alkanolrest $-OR_2$ und B einen Rest $R_6$ bedeutet.

Je nach den Reaktionsverhältnissen und den Substituenten liegen die Verbindungen als E und Z-Isomere Oxime vor.

Die Herstellung der neuen Cyclohexenoncarbonsäurederivate der Formel I erfolgt in an sich bekannter Weise durch Umsetzen eines 3,5-Cyclohexandioncarbonsäurederivates, respektive eines 3-Hydroxy-5-oxo-cyclohexencarbonsäurederivates der Formel II

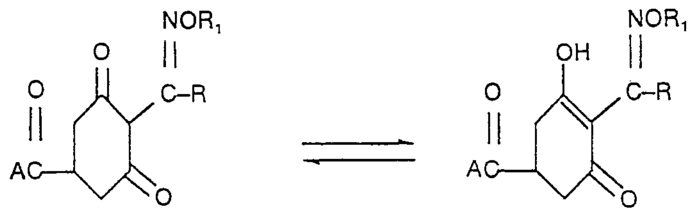

(II)

worin A, R und $R_1$ die oben gegebene Bedeutung haben, in einem inerten organischen Lösungsmittel in Gegenwart einer Base mit einem reaktionsfähigen Ester einer Verbindung der Formel

BZ (III),

worin B die im Anspruch 1 gegebene Bedeutung hat, und Z eine Abgangsgruppe bedeutet.

Die Verbindungen der Formel BZ sind entweder Alkylhalogenide, Carbon- oder Sulfonsäurederivate. Als reaktionsfähige Säurederivate kommen vor allem deren Halogenide in Frage aber auch Ester resp. gemischte Anhydride mit niederen Fettsäuren, wie z.B. dem Acetat, Säureanhydride der Formel BCOOCOB oder falls B einen Rest $R_6$ bedeutet auch dessen Methyl-, Tolyl- oder Benzolsulfonsäurereste.

Als inerte organische Lösungsmittel für diese Umsetzung kommen vor allem Aromaten wie Benzol, Toluol, sowie Halogenwasserstoffe wie Chloroform, Dichloräthan, Tetrachlorkohlenstoff oder auch Ketone und Ester wie z.B. Aceton oder Äthylacetat in Frage.

Die Reaktionstemperaturen liegen bei Raumtemperatur und dem Siedepunkt des Reaktionsgemisches. Während der Zugabe von Säurechlorid ist möglicherweise eine Kühlung des Reaktionsgefässes angezeigt.

Als Basen kommen die organischen wie anorganischen in Betracht. Beispiele sind Pyridin, 4-Aminopyridin, Kollidin, Triäthylamin, Ammonium-, Natrium-, Kalium- oder Calciumcarbonat oder die entsprechenden Bicarbonate.

Die als Ausgangsprodukte benötigten Cyclohexandioncarbonsäurederivate werden ausgehend von 3,5-Dihydroxybenzoesäure erhalten. Die Benzoesäure wird zuerst mit Wasserstoff in Gegenwart von Raney-Nickel hydriert, dann am Säurerest verestert oder amidiert, wobei die Ketogruppe gegebenenfalls geschützt werden muß, z.B. als Enoläther oder als Enamin, siehe J. Am. Chem. Soc. 78, 4405 (1956).

Man kann beispielsweise auch einen 3,5-Dihydroxybenzoesäureester oder ein Amid hydrieren um zum selben 3,5-Dioxocyclohexancarbonsäurederivat zu gelangen, s. Arch Pham. 3207, 577 (1974)

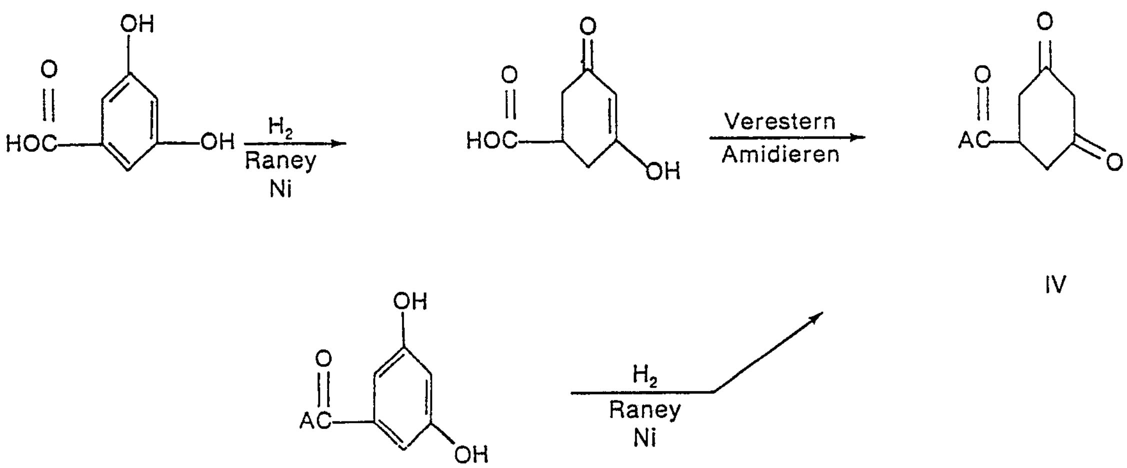


Das so erhaltene 3,5-Dicyclohexancarbonsäurederivat der Formel IV wird dann in einem inerten organischen Lösungsmittel, in Gegenwart einer Base, mit einem Säurehalogenid der Formel V HalCOR umgesetzt, worin R die oben gegebene Bedeutung hat, das erhaltene Produkt isoliert und gegebenenfalls weiter in einem inerten mit Wasser nicht mischbaren Lösungsmittel bei Siedetemperatur unter wasserabspaltenden Bedingungen mit einem Hydroxylamin der Formel VI, $HONHR_1$, worin $R_1$ die oben gegebene Bedeutung hat, umgesetzt entsprechend dem Schema

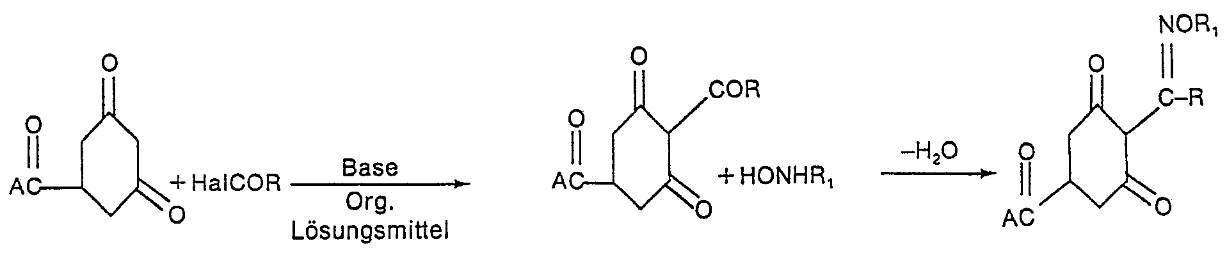


IV V VI II

Als Lösungsmittel für diese Umsetzungen kommen auch hier Aromaten wie Halogenwasserstoffe in Frage. Es können die obgenannten Basen verwendet werden.

Die Reaktionstemperaturen liegen bei Raumtemperatur und dem Siedepunkt des Reaktionsgemisches. Während der Zugabe des Säurechlorides ist eine Kühlung des Reaktionsgefässes angezeigt.

Als Säurehalogenide der Formel V kommen hauptsächlich Essigsäurechlorid, Propionsäurechlorid, Buttersäurechlorid, Valeriansäurechlorid, 3-Methoxypropionsäurechlorid, 2-Chlorpropionsäurechlorid, Cyclopropansäurechlorid oder Cyclohexansäurechlorid, wie auch die entsprechenden Bromide in Frage. Die Herstellung dieser Ausgangsprodukte ist in der schweizerischen Patentanmeldung Nr. 6747/83-0, diejenige einer solchen Verbindung im Beispiel 1a–1c näher beschrieben.

Die Verbindungen der Formel I haben herbizide und das Pflanzenwachstum regulierende Wirkung, sie eignen sich z.B. zur selektiven Bekämpfung von Gräsern in Nutzpflanzenkulturen.

Bei geringen Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Zuckerrohr, Getreide, Baumwolle, Soja, Mais und Reis befähigen. Es werden dabei teilweise auch Unkräuter geschädigt, welchen bisher nur mit Totalherbiziden beizukommen war.

Die Verbindungen der Formel I haben außerdem starke pflanzenwuchsregulierende Eigenschaften, die sich in einer Ertragssteigerung von Kulturpflanzen oder Erntegut auswirken kann. Viele Verbindungen der Formel I zeigen darüberhinaus eine konzentrationsabhängige pflanzenwuchshemmende Wirkung. Es werden sowohl Monokotyledonen als auch Dikotyledonen in ihrem Wachstum beeinträchtigt.

So können z.B. die in der Landwirtschaft in tropischen Gegenden häufig als «cover crops» (Bodendecker) angepflanzten Leguminosen durch die Verbindungen der Formel I in ihrem Wachstum selektiv gehemmt werden, so daß zwar die Bodenerosion zwischen den Kulturpflanzen verhindert wird, die «cover crops» jedoch nicht zur Konkurrenz für die Kultur werden können.

Eine Hemmung des vegetativen Wachstums erlaubt bei vielen Kulturpflanzen eine dichtere Anpflanzung der Kultur, so daß ein Mehrertrag, bezogen auf die Bodenfläche, erzielt werden kann.

Ein weiterer Mechanismus der Ertragssteigerung mit Wachstumshemmern beruht darauf, daß die Nährstoffe in stärkerem Masse der Blüten- und Fruchtbildung zugute kommen, während das vegetative Wachstum eingeschränkt wird.

Bei monokotylen Pflanzen, z.B. Gräsern oder auch Kulturpflanzen wie Getreide, ist eine Hemmung des vegetativen Wachstums manchmal gewünscht und vorteilhaft. Eine derartige Wachstumshemmung ist unter anderem bei Gräsern von wirtschaftlichem Interesse, denn dadurch kann z.B. die Häufigkeit der Grasschnitte in Ziergärten, Park- und Sportanlagen oder an Straßenrändern reduziert werden. Von Bedeutung ist auch die Hemmung des Wuchses von krautigen und holzigen Pflanzen an Straßenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens («Lagerns») der Pflanzen vor der Ernte verringert oder vollkommen beseitigt. Außerdem können Wachstumsregulatoren bei Getreide eine Hahnverkürzung hervorrufen, die ebenfalls dem Lagern entgegenwirkt.

Weiter eignen sich die Verbindungen der Formel I, um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei größeren Aufwandmengen werden alle getesteten Pflanzen in ihrer Entwicklung so geschädigt, daß sie absterben.

Die Erfindung betrifft auch herbizide und pflanzenwuchsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I in unveränderter Form oder vorzugsweise als Mittel zusammen mit den in der Formulierungstechnik üblichen Hilfsmittel eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Gießen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnußöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder

hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$–$C_{22}$), wie z.B. die Na- oder K-Salze der Öl- oder Stearinsäure, oder von natürlichen Fettsäuregemischen, die z.B. aus Kokosnuß- oder Talgöl gewonnen werden können, genannt. Ferner sind auch die Fettsäure-methyltaurinsalze zu erwähnen.

Häufig werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2-Sulfonsäuregruppen und einen Fettsäurerest mit 8–22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4–14)-Äthylenoxid-Adduktes und Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykolääthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

«Mc Cutcheon's Detergents and Emulsifiers Annual» MC

Publishing Corp., Ridgewood, New Jersey, 1979.

J. and M. Ash, «Encyclopedia of Surfactants» Vol. I–III.

Chemical Publishing Co., Inc. New York, 1980/81

H. Stache «Tensid-Taschenbuch» C. Hanser Verlag München, Wien 1980

Die Wirkstoffzubereitungen enthalten in der Regel 0,1 bis 95%, insbesondere 0,1 bis 80%, Wirkstoff der Formel I, 1 bis 99,9% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent).

Emulgierbare Konzentrate
Aktiver Wirkstoff:
1 bis 20%, bevorzugt 5 bis 10%
oberflächenaktive Mittel:
5 bis 30%, bevorzugt 10 bis 20%
flüssiges Trägermittel:
50 bis 94%, bevorzugt 70 bis 85%.

Stäube
Aktiver Wirkstoff:
0,1 bis 10%, vorzugsweise 0,1 bis 1%
festes Trägermittel:
99,9 bis 90%, vorzugsweise 99,9 bis 99%.

Suspensions-Konzentrat
Aktiver Wirkstoff:
5 bis 75%, vorzugsweise 10 bis 50%
Wasser:
94 bis 25%, vorzugsweise 90 bis 30%

oberflächenaktives Mittel:
1 bis 40%, vorzugsweise 2 bis 30%.
Benetzbare Pulver
Aktiver Wirkstoff:
0,5 bis 90%, vorzugsweise 1 bis 80%
oberflächenaktives Mittel:
0,5 bis 20%, vorzugsweise 1 bis 15%
festes Trägermittel:
5 bis 95%, vorzugsweise 15 bis 90%.
Granulate
Aktiver Wirkstoff:
0,5 bis 30%, vorzugsweise 3 bis 15%
festes Trägermittel:
99,5 bis 70%, vorzugsweise 97 bis 85%.

Während als Handelswerte eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,001 bis 10 kg/ha, vorzugsweise 0,025 bis 5 kg/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

Herstellungsbeispiele: Beispiel 1:
Herstellung von 4-(1-Äthoxyaminobutyliden)-3,5-cyclohexandion-carbonsäure-dimethylamid
(Zwischenprodukt)

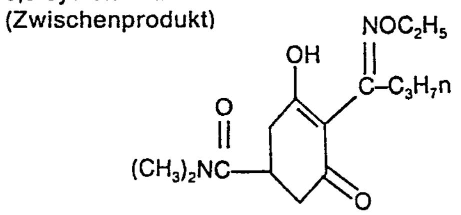

a) Zu einer Lösung von 15,6 g Cyclohexandioncarbonsäure in 50 ml Pyridin tropft man unter Rühren 18,4 ml Dimethylcarbamoylchlorid und rührt anschließend 12 Stunden bei Raumtemperatur und 2 Stunden bei Siedetemperatur unter Rückfluß weiter. Nach dem Abkühlen nimmt man das Reaktionsgemisch in 400 ml Äthylacetat auf, wäscht die organische Phase viermal mit Salzsole, trocknet über Magnesiumsulfat und engt sie ein. Der Rückstand besteht aus rohem 3-(N,N-Dimethylcarbamoyl)-5-oxo-cyclohex-(3)-en-carbonsäure-dimethylamid.

Dieser wird in 300 ml Tetrahydrofuran gelöst, mit 8 ml konzentrierter Salzsäure versetzt und 2 Stunden bei Raumtemperatur gerührt. Das Reaktionsgemisch wird dann mit Salzsole gewaschen, über Magnesiumsulfat getrocknet und eingeengt. Es verbleiben 13,2 g 3,5-Cyclohexandioncarbonsäure-dimethylamid als harzartige Substanz, die nach Reinigung durch Chromatographie mit Hexan/Äther über eine Silikagelkolonne kristallin anfällt. Schmelzpunkt 152–155 °C.

b) Die 13,2 g 3,5-Cyclohexandioncarbonsäure-dimethylamid werden zusammen mit 6,9 ml Pyridin in 100 ml Äthylenchlorid gelöst. Dann tropft man unter Rühren 8,5 ml Buttersäurechlorid zu dieser Lösung, was eine leicht exotherme Reaktion hervorruft. Die entstandene gelbe Suspension wird während 14 Stunden bei Raumtemperatur gerührt, dann mit 1 N Salzsäure und Salzsole gewaschen, über Magnesiumsulfat getrocknet und eingeengt. Der Rückstand wird in 100 ml Dichloräthan gelöst und mit 0,5 g 4-Dimethylaminopyridin und 0,1 ml Buttersäurechlorid während 2 Stunden am Rückfluß gekocht. Nach dem Erkalten wird das Reaktionsgemisch mit 20 ml 1 n, mit Kochsalz gesättigter Salzsäure gewaschen, über Magnesiumsulfat getrocknet und eingeengt. Der Rückstand wird zur Reinigung über eine Silikagelkolonne mit Äthylenacetat als Laufmittel chromatographiert. Man erhält so 7,8 g 4-Butyryl-3,5-cyclohexandioncarbonsäure-dimethylamid als helles Öl.

c) Ein Gemisch von 4,3 g des oben erhaltenen 4-Butyryl-3,5-cyclohexandioncarbonsäure-dimethylamides,
1,9 g Äthoxyamin-Hydrochlorid, 1,4 g Kaliumcarbonat in 50 ml Chloroform und 5 ml Methanol wird während 24 Stunden bei Raumtemperatur gerührt. Das Reaktionsgemisch wird dann mit 1 N Salzsäure gewaschen, über Magnesiumsulfat getrocknet und eingeengt. Der Rückstand ist ein Öl, welches durch Chromatographie über eine Silikagelsäure mit Äthylacetat als Laufmittel gereinigt wird. Nach Verdampfen des Lösungsmittels verbleibt ein Öl, das beim Stehen kristallisiert. Man erhält so 2 g Titelsubstanz vom Schmelzpunkt 54–58°.

In analoger Weise zu diesem Beispiel werden die folgenden, als Zwischenprodukte benötigten 3,5-Cyclohexandion-1-carbonsäurederivate der Formel II hergestellt.

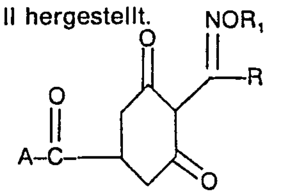

(II)

Tabelle 1

| Nr. | A | R | $R_1$ | physik. Daten |
|---|---|---|---|---|
| 1.001 | $OCH_3$ | $C_3H_7n$ | $C_2H_5$ | $n_D^{27}$ 1.5100 |
| 1.002 | $OC_2H_5$ | $C_5H_7n$ | $C_2H_5$ | $n_D^{27}$ 1.5002 |
| 1.003 | $OC_2H_5$ | $C_3H_7n$ | $CH_2CH=CH_2$ | $n_D^{27}$ 1.5112 |
| 1.004 | $OC_4H_9$ iso | $C_3H_7n$ | $C_2H_5$ | $n_D^{27}$ 1.4929 |
| 1.005 | $OC_4H_9$ iso | $C_3H_7n$ | $CH_2CH=CH_2$ | $n_D^{27}$ 1.4989 |
| 1.006 | $OCH_2SCH_3$ | $C_3H_7n$ | $C_2H_5$ | $n_D^{25}$ 1.5198 |
| 1.007 | $OCH_2SCH_3$ | $C_3H_7n$ | $CH_2-C\equiv CH$ | |
| 1.008 | $OC_3H_7$ iso | $C_3H_7n$ | $C_2H_5$ | $n_D^{25}$ 1.5003 |

Tabelle 1 (Fortsetzung)

| Nr. | A | R | $R_1$ | physik. Daten |
|---|---|---|---|---|
| 1.009 | $OC_3H_7$ iso | $C_3H_7$n | $CH_2–CH=CH_2$ | $n_D^{25}$ 1.5088 |
| 1.010 | $OC_3H_7$ iso | $C_3H_7$n | $CH_2CCl=CH_2$ | |
| 1.011 | $OC_2H_4SCH_3$ | $C_3H_7$n | $C_2H_5$ | |
| 1.012 | $OC_2H_4SCH_3$ | $C_3H_7$n | $CH_2–CH=CH_2$ | |
| 1.013 | $OC_2H_4SCH_3$ | $C_3H_7$n | $C_2H_4Cl$ | |
| 1.014 | $OC_2H_4OCH_3$ | $C_3H_7$n | $CH_3$ | |
| 1.015 | $OC_2H_4OCH_3$ | $C_3H_7$n | $C_2H_5$ | |
| 1.016 | $OC_3H_6Cl$ | $C_3H_7$n | $C_2H_5$ | |
| 1.017 | $OCH_2$ 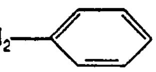 | $C_3H_7$n | $C_2H_5$ | |
| 1.018 | $OCH_2$ 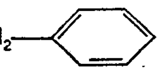 | $C_3H_7$n | $CH_2–CH=CH_2$ | |
| 1.019 | $OCH_2$ 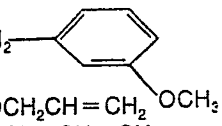 | $C_3H_7$n | $C_6H_{13}$n | |
| 1.020 | $OCH_2CH=CH_2$ | $C_3H_7$n | $C_6H_{13}$n | |
| 1.021 | $OCH_2–CH=CH_2$ | $C_3H_7$n | $CH_3$ | |
| 1.022 | $OC_2H_4Cl$ | $C_3H_7$n | $CH_2–C\equiv CH_2$ | |
| 1.023 | $OCH_2CCl=CH_2$ | $C_3H_7$n | $C_2H_4Cl$ | |
| 1.024 | $OCH_2–C\equiv CH$ | $C_3H_7$n | $C_6H_{13}$ | |
| 1.025 | $OCH_3$ | $C_6H_{13}$n | $C_2H_5$ | |
| 1.026 | $OC_2H_5$ | cyclo-propyl | $CH_2CH=CH_2$ | |
| 1.027 | $OCH_3$ | cyclo-hexyl | $C_2H_5$ | |
| 1.028 | $OC_6H_{13}$n | $CH_3$ | $C_4H_9$n | |
| 1.029 | $OCH_2SCH_3$ | $CH_3$ | $C_4H_9$ sec | |
| 1.030 | $OC_2H_5$ | $CH_2SCH_3$ | $C_2H_5$ | |
| 1.031 | $OC_2H_5$ | $C_2H_4OCH_3$ | $CH_2–C\equiv CH$ | |
| 1.032 | $OC_4H_9$ tert. | $CHCl–CH_3$ | $C_2H_5$ | |
| 1.033 | $NH_2$ | $C_3H_7$n | $C_2H_5$ | |
| 1.034 | $NH_2$ | $C_3H_7$n | $CH_2–CH=CH_2$ | Smp. 127–129° |
| 1.035 | $NH_2$ | $C_2H_5$ | $C_2H_5$ | |
| 1.036 | $NH_2$ | $C_2H_5$ | $CH_2–Cl=CH_2$ | |
| 1.037 | $N(CH_3)_2$ | $C_3H_7$n | $C_2H_5$ | Smp. 54–58° |
| 1.038 | $N(CH_3)_2$ | $C_3H_7$i | $CH_2–CH=CH_2$ | Smp. 59–65° |
| 1.039 | $N(CH_3)CH_2$ | $C_3H_7$i | $CH_3$ | |
| 1.040 | $N(CH_3)_2$ | $C_3H_7$i | $C_3H_6Br$ | |
| 1.041 | $NHC_4H_9$ iso | $C_3H_7$n | $C_2H_5$ | Smp. 88–90° |
| 1.042 | $NHC_4H_9$ iso | $C_3H_7$n | $CH_3$ | |
| 1.043 | $NHC_4H_9$ iso | $C_3H_7$n | $CH_2CH=CH_2$ | Smp. 100–102° |
| 1.044 | $NHC_4H_9$ iso | $CH_3$ | $CH_2C\equiv CH$ | |
| 1.045 | $N(CH_2–CH=CH_2)_2$ | cyclo-propyl | $C_2H_5$ | |
| 1.046 | $NHCH_2–C\equiv CH$ | $CH_2OCH_3$ | $C_5H_{11}$ sec | |
| 1.047 | $NHCH_2$ 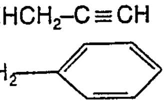 | $C_3H_7$n | $C_2H_5$ | Smp. 116–119° |
| 1.048 | $NHCH_2$ 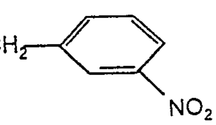 | $C_2H_4Cl$ | $C_2H_4F$ | |
| 1.049 | $N(CH_3)CH_2$ 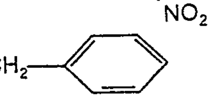 | $C_2H_4OC_2H_4$ | $CH_2CF_3$ | |
| 1.050 | $NHC_2H_4OCH_3$ | $C_3H_7$n | $C_2H_5$ | |
| 1.051 | $NHC_2H_4SCH_3$ | $C_4H_9$ iso | $CH_3$ | |
| 1.052 | piperidino | $C_3H_7$i | $CH_2–CH=CH_2$ | |
| 1.053 | morpholino | $C_6H_{13}$ | $C_2H_5$ | |

Tabelle 1 (Fortsetzung)

| Nr. | A | R | $R_1$ | physik. Daten |
|---|---|---|---|---|
| 1.054 | $N(CH_3)OCH_3$ | $C_3H_7n$ | $C_2H_5$ | $n_D^{25}$ 1.5122 |
| 1.055 | $N(CH_3)OCH_3$ | $C_3H_7n$ | $CH_2CH=CH_2$ | |
| 1.056 | $N(CH_3)OCH_3$ | $C_3H_7n$ | $CH_2CH=CHCl$ | Öl |
| 1.057 | $N(C_2H_5)_2$ | $C_3H_7n$ | $C_2H_5$ | Wachs |
| 1.058 | $N(C_2H_5)_2$ | $C_3H_7n$ | $CH_2CH=CH_2$ | |
| 1.059 | $N(CH_3)_2$ | cyclo-propyl | $C_2H_5$ | |
| 1.060 | 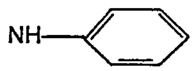 | cyclo-propyl | $CH_2CH=CH_2$ | |
| 1.061 | $N(CH_3)OCH_3$ | $C_2H_5$ | $C_2H_5$ | |
| 1.062 | 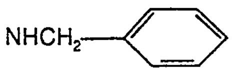 | $C_4H_9$ iso | $C_2H_5$ | |
| 1.063 | 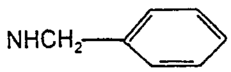 | cyclo-propyl | $CH_2CH=CH_2$ | |
| 1.064 | 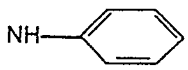 | $C_3H_7n$ | $C_2H_5$ | Smp. 123–126° |
| 1.065 | 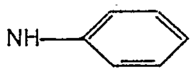 | $C_3H_7n$ | $CH_2CH=CH_2$ | Wachs |
| 1.066 | 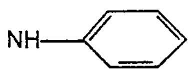 | $CH_3$ | $CH_2CH=CH_2$ | |
| 1.067 | 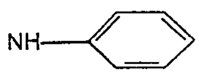 | $C_6H_{13}n$ | $C_2H_5$ | |
| 1.068 | 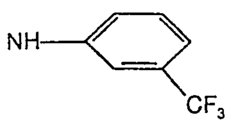 | $C_3H_7n$ | $C_2H_5$ | |
| 1.069 | 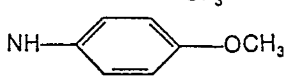 | $C_6H_{13}n$ | $C_2H_5$ | |
| 1.070 | 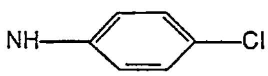 | $C_6H_{13}$ | $CH_2CH=CH_2$ | |
| 1.071 | 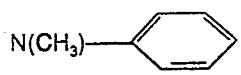 | $C_6H_{13}n$ | $CH_2CH=CH_2$ | |
| 1.072 | Cyclopropyl-amino | $C_6H_{13}n$ | $C_2H_5$ | |
| 1.073 | $NHC_6H_{13}n$ | $CH_3$ | $C_2H_5$ | |
| 1.074 | 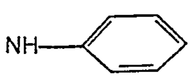 | $C_3H_7n$ | $CH_2CH=CHCl$ | |
| 1.075 | $N(CH_3)_2$ | $C_3H_7n$ | $CH_2CH=CHCl$ | |
| 1.076 | 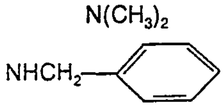 | $C_3H_7n$ | $CH_2CH=CHCl$ | |
| 1.077 | $NHC_2H_4SCH_3$ | $C_3H_7n$ | $C_2H_5$ | Smp. 61–67° |
| 1.078 | $NHC_2H_4SCH_3$ | $C_3H_7n$ | $CH_2CH=CH_2$ | |
| 1.079 | $NHC_2H_4SCH_3$ | $C_3H_7n$ | $CH_2CH=CHCl$ | |
| 1.080 | $N(CH_3)_2$ | cyclo-propyl | $CH_2CH=CH_2$ | |

Tabelle 1 (Fortsetzung)

| Nr. | A | R | $R_1$ | physik. Daten |
|---|---|---|---|---|
| 1.081 | $N(CH_3)_2$ | $C_3H_7n$ | $C_4H_9n$ | Smp. 67–70° |
| 1.082 | $N(CH_3)_2$ | $C_3H_7n$ | $C_6H_{13}n$ | Smp. 65–68° |
| 1.083 | $N(CH_3)_2$ | $C_2H_5$ | $C_2H_5$ | Öl |
| 1.084 | $N(CH_3)_2$ | $C_2H_5$ | $CH_2CH=CH_2$ | Smp. 82–83° |
| 1.085 | $N(CH_3)_2$ | $C_6H_{13}n$ | $C_2H_5$ | |
| 1.086 | $N(CH_3)_2$ | $C_5H_{11}n$ | $CH_2CH=CH_2$ | Smp. 64–66° |
| 1.087 | 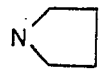 | $C_3H_7n$ | $C_2H_5$ | Smp. 77–85° |
| 1.088 | 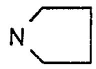 | $C_8H_7n$ | $C_2H_5$ | |
| 1.089 | 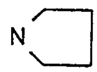 | $C_3H_7n$ | $CH_2CH=CHCl$ | |
| 1.090 | $NHCH_3$ | $C_3H_7n$ | $C_2H_5$ | Smp. 138–140° |
| 1.091 | $NHCH_3$ | $C_3H_7n$ | $CH_2CH=CH_2$ | |
| 1.092 | $NHCH_3$ | $C_3H_7n$ | $CH_3$ | Smp. 139–141° |
| 1.093 | 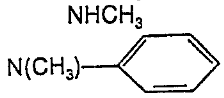 | $C_3H_7n$ | $CH_2CH=CHCl$ | Smp. 52–56° |
| 1.094 | 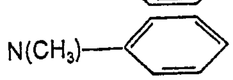 | $C_3H_7n$ | $C_2H_5$ | Smp. 93–95° |
| 1.095 | $N(CH_2CH=CH_2)_2$ | $C_3H_7n$ | $C_2H_5$ | |
| 1.096 | $N(CH_2CH=CH_2)_2$ | $C_3H_7n$ | $CH_2CH=CH_2$ | Öl |
| 1.097 | $N(CH_2CH=CH_2)_2$ | $C_3H_7n$ | $CH_2CH=CHCl$ | Öl |
| 1.098 | $N(CH_2CH=CH_2)_2$ | $C_3H_7n$ | $CH_3$ | |
| 1.099 | $NHCH_3$ | $C_3H_7n$ | $C_6H_{13}n$ | |
| 1.100 | $NHCH_3$ | $C_3H_7n$ | $C_4H_9n$ | |
| 1.101 | $NHCH_3$ | $C_5H_{11}n$ | $CH_2CH=CH_2$ | |
| 1.102 | $NHCH_3$ | $C_6H_{13}n$ | $C_4H_9n$ | |
| 1.103 | $NHC_2H_4SCH_3$ | $C_2H_5$ | $CH_2CH=CH_2$ | |
| 1.104 | $NHC_2H_4SC_3H_7i$ | $C_3H_7n$ | $CH_2CH=CH_2$ | |
| 1.105 | $NHC_2H_4SC_3H_7i$ | $C_3H_7n$ | $C_4H_9n$ | |
| 1.106 | $N(CH_3)C_2H_5$ | $C_3H_7n$ | $CH_2CH=CH_2$ | |
| 1.107 | $N(CH_3)C_2H_5$ | $C_3H_7n$ | $C_4H_9n$ | |
| 1.108 | $N(CH_3)C_2H_5$ | $C_3H_7n$ | $CH_2CH=CHCl$ | |
| 1.109 | $N(CH_3)_2$ | $C_5H_{11}n$ | $C_2H_5$ | |
| 1.110 | $OCH_3$ | $CH_3$ | $C_2H_5$ | $n_n^{30}$ 1.5077 |
| 1.111 | $N(CH_3)_2$ | $C_3H_7n$ | $CH_2CH=CHCl$ | Öl |
| 1.112 | $OC_2H_5$ | $C_3H_7n$ | $C_4H_9n$ | Öl |
| 1.113 | $N(CH_3)_2$ | $C_2H_5$ | $C_4H_9n$ | Smp. 59–63° |
| 1.114 | 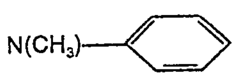 | $C_3H_7n$ | $C_4H_9i$ | Öl |
| 1.115 | $N(CH_3)_2$ | $C_3H_7n$ | $C_4H_9i$ | Öl |
| 1.116 | OH | $C_3H_7n$ | $C_4H_9n$ | Öl |
| 1.117 | $N(CH_3)_2$ | $C_2H_5$ | $CH_2CH=CHCl$ | Smp. 69–73° |
| 1.118 | $N(C_2H_5)_2$ | $C_2H_5$ | $C_2H_5$ | Smp. 90–93° |
| 1.119 | $N(C_2H_5)_2$ | $C_2H_5$ | $CH_2CH=CH_2$ | Smp. 73–76° |
| 1.120 | 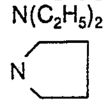 | $C_2H_5$ | $C_2H_5$ | Smp. 58–60° |
| 1.121 | 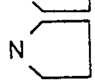 | $C_2H_5$ | $CH_2CH=CH_2$ | Smp. 70–72° |
| 1.122 | $N(CH_3)_2$ | $C_3H_7n$ | $CH_2C(CH_3=CH_2$ | Smp. 55–57° |
| 1.123 | $N(CH_3)_2$ | $CH_3$ | $CH_2CH=CHCl$ | Wachs |

Beispiel 2:
Herstellung von 3-Acetoxy-4-(1-Äthoximino-butyl)-5-oxo-cyclohex-3-en-carbonsäure-dimethylamid

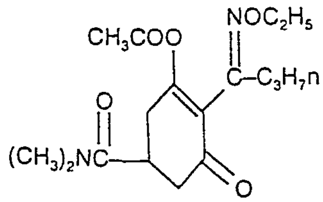

Zu einer Lösung von 5 g
4-(1-Äthoxyaminobutyliden)-3,5-cyclohexandioncarbonsäure-dimethylamid
in 70 ml Aceton tropft man unter Rühren 8,5 ml 2 M wäßrige Natronlauge. Nach 20 Minuten wird die Lösung am Rotationsverdampfer zur Trockne eingeengt. Der Rückstand wird in 70 ml Aceton aufgeschlämmt und nach Zugabe von 1,22 ml Acetylchlorid zwei Stunden bei Raumtemperatur gerührt. Die Reaktionslösung wird unter Vakuum eingeengt, der Rückstand in Acetylacetat aufgenommen, mit Natronlauge und gesättigter Kochsalzsole gewaschen, getrocknet und eingeengt. Man erhält so 2,3 g Titelprodukt als Öl.

Beispiel 3:
Herstellung von 3-Benzoyloxy-4-(1-äthoximino-butyl)-5-oxo-cyclohex-3-en-carbonsäure-dimethylamid

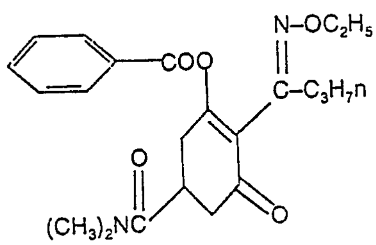

Zu einer Lösung von 3,9 g
4-(1-Äthoximino-butyliden)-3,5-cyclohexandioncarbonsäure-dimethylamid
und 1,55 ml Benzoylchlorid in 80 ml Aceton gibt man 1,8 g Kaliumcarbonat und rührt während 15 Stunden bei Raumtemperatur. Dann wird die Lösung mit 80 ml Acetylacetat verdünnt, filtriert und am Rotationsverdampfer bei 45 °C unter Vakuum eingeengt. Es verbleiben 4,6 g Öl, als Rückstand, die gewünschte Titelverbindung.

Beispiel 4:
Herstellung von 4-(1-Äthoximino-butyl)-3-methoxy-5-oxo-cyclohex-3-en-carbonsäure-dimethylamid

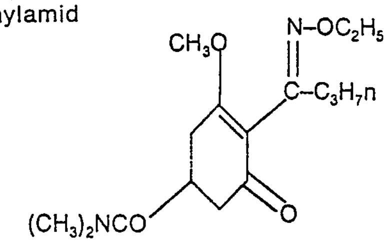

Zu einer Lösung von 5 g
4-(1-Äthoxyaminobutyliden)-3,5-cyclohexandioncarbonsäure-dimethylamid
und 0,85 ml Dimethylsulfat in 70 ml Aceton gibt man 2,4 g Kaliumcarbonat und rührt 15 Stunden bei Raumtemperatur. Dann wird das Reaktionsgemisch mit 70 ml Chloroform verdünnt, filtriert und am Rotationsverdampfer eingeengt. Das zurückbleibende Öl wird durch Chromatographie über eine Silikagelsäule in seine Isomere aufgeteilt. Man erhält so 1,6 g des einen (Trans)-Isomers mit Schmelzpunkt 188–194° und 0,5 g eines zweiten (CIS)-Isomers, welches bei 147–150° schmilzt.

Die Herstellung des Ausgangsmaterials ist im Beispiel 1a–1c beschrieben.

In analoger Weise zu den Beispielen 2 bis 4 werden die folgenden Verbindungen der Tabelle 2 ausgehend von Verbindungen der Tabelle 1, hergestellt.

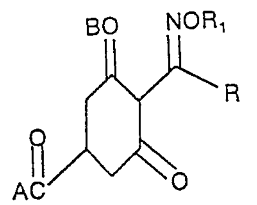

Tabelle 2

| Nr. | A | R | $R_1$ | B | |
|---|---|---|---|---|---|
| 2.01 | $-OCH_3$ | $C_3H_7(n)$ | $-C_2H_5$ | $CH_3$ | |
| 2.02 | $-OCH_3$ | $C_3H_7(n)$ | $-C_2H_5$ | $COCH_3$ | |
| 2.03 | $-OCH_3$ | $C_3H_7(n)$ | $-C_2H_5$ | Benzoyl | |
| 2.04 | $-OCH_3$ | $C_3H_7(n)$ | $-C_2H_5$ | Benzyl | |
| 2.05 | $-OCH_3$ | $C_3H_7(n)$ | $-C_2H_5$ | p-Tolylsulfonyl | |
| 2.06 | $-OCH_3$ | $C_3H_7(n)$ | $-CH_2CH=CH_2$ | $-COCH_3$ | |
| 2.07 | $-OCH_3$ | $C_3H_7(n)$ | $-CH_2CH=CH_2$ | $-SO_2CH_3$ | |
| 2.08 | $-OCH_3$ | $C_3H_7(n)$ | $-CH_2CH=CH_2$ | $-C_2H_5$ | |
| 2.09 | $OC_3H_7(i)$ | $C_3H_7(n)$ | $-C_2H_5$ | $-COCH_2$ | |
| 2.10 | $OC_3H_7(i)$ | $C_3H_7(n)$ | $-C_2H_5$ | Benzoyl | |
| 2.11 | $OC_3H_7(i)$ | $C_3H_7(n)$ | $-CH_2CH=CH_2$ | 3-Chlorbenzoyl | |
| 2.12 | $OC_3H_7(i)$ | $C_3H_7(n)$ | $-CH_2CH=CH_2$ | Phenylsulfonyl | |
| 2.13 | $OC_3H_7(i)$ | $C_3H_7(n)$ | $-CH_2CH=CH_2$ | $-CH_2-CH=CH_2$ | |
| 2.14 | $N(CH_3)_2$ | $C_3H_7(n)$ | $-C_2H_5$ | $-COCH_3$ | Beispiel 2 |
| 2.15 | $N(CH_3)_2$ | $C_3H_7(n)$ | $-C_2H_5$ | Benzoyl | Beispiel 3 |
| 2.16 | $N(CH_3)_2$ | $C_3H_7(n)$ | $-C_2H_5$ | $-CH_3$ | Beispiel 4 |
| 2.17 | $N(CH_3)_2$ | $C_3H_7(n)$ | $-C_2H_5$ | p-Tolylsulfonyl | |
| 2.18 | $N(CH_3)_2$ | $C_3H_7(n)$ | $-CH_2CH=CH_2$ | $-COCH_3$ | |
| 2.19 | $N(CH_3)_2$ | $C_3H_7(n)$ | $-CH_2CH=CH_2$ | Benzoyl | |

Tabelle 2 (Fortsetzung)

| Nr. | A | R | $R_1$ | B | |
|---|---|---|---|---|---|
| 2.20 | $N(CH_3)_2$ | $C_3H_7(n)$ | $-CH_2CH=CH_2$ | $-CH_3$ | |
| 2.21 | $N(CH_3)_2$ | $C_3H_7(n)$ | $-CH_2CH=CH_2$ | p-Tolylsulfonyl | |
| 2.22 | $N(CH_3)_2$ | $C_3H_7(n)$ | $-CH_2CH=CHCl$ | n-Butyryl | |
| 2.23 | $N(CH_3)_2$ | $C_3H_7(n)$ | $-CH_2CH=CHCl$ | p-Chlorbenzoyl | |
| 2.24 | $N(CH_3)_2$ | $C_2H_5$ | $-CH_2CH=CHCl$ | $-COCH_3$ | |
| 2.25 | $N(CH_3)_2$ | $C_2H_5$ | $-CH_2CH=CHCl$ | n-Butyryl | |
| 2.26 | $N(CH_3)_2$ | $C_2H_5$ | $-CH_2CH\equiv CHCl$ | $-CH_2-C\equiv CH$ | |
| 2.27 | $N(CH_3)_2$ | $C_6H_{13}$ | $-CH_3$ | $-CH_3$ | |
| 2.28 | $N(CH_3)_2$ | $C_6H_{13}$ | $-C_2H_5$ | $-COCH=CH_2$ | |
| 2.29 | $NH_2$ | $C_3H_7(n)$ | $-CH_2CH=CH_2$ | $-COCH_3$ | |
| 2.30 | $NH_2$ | $C_3H_7(n)$ | $-CH_2CH=CH_2$ | $-COCH_2CH_3$ | |
| 2.31 | $NH_2$ | $C_3H_7(n)$ | $-CH_2CH=CH_2$ | Benzoyl | Harz |
| 2.32 | $NH_2$ | $C_3H_7(n)$ | $-CH_2CH=CH_2$ | p-Tolylsulfonyl | |
| 2.33 | $NH_2$ | $C_3H_7(n)$ | $-CH_2CH=CH_2$ | $-CH_3$ | |
| 2.34 | $NH_2$ | $C_3H_7(n)$ | $-CH_2CH=CH_2$ | Benzyl | |
| 2.35 | $NH_2$ | $C_3H_7(n)$ | $C_2H_5$ | $-COCH_3$ | |
| 2.36 | $NH_2$ | $C_3H_7(n)$ | $-C_2H_5$ | $-SO_2CH_3$ | |
| 2.37 | $NH_2$ | $C_3H_7(n)$ | $-C_2H_5$ | p-Anisoyl | |
| 2.38 | $NH_2$ | $C_3H_7(n)$ | $-C_2H_5$ | $-CH_2-CH_3$ | |
| 2.39 | $NHCH_3$ | $C_3H_7(n)$ | $-CH_2CH=CH_2$ | $-COCH_3$ | |
| 2.40 | $NHCH_3$ | $C_3H_7(n)$ | $-CH_2CH=CH_2$ | Benzoyl | |
| 2.41 | $NHCH_3$ | $C_3H_7(n)$ | $-CH_2CH=CH_2$ | Benzyl | |
| 2.42 | $NHCH_3$ | $C_3H_7(n)$ | $-C_2H_5$ | $-CH_3$ | |
| 2.43 | $NHCH_3$ | $C_3H_7(n)$ | $-C_2H_5$ | Benzoyl | |
| 2.44 | $NHCH_3$ | $C_3H_7(n)$ | $-C_2H_5$ | $-SO_2CH_3$ | |
| 2.45 | $NHCH_3$ | $C_3H_7(n)$ | $-C_4H_9(n)$ | Benzoyl | |
| 2.46 | $NHCH_3$ | $C_3H_7(n)$ | $-C_4H_9(n)$ | $-CH_3$ | |
| 2.47 | Anilino | $C_3H_7(n)$ | $-C_2H_5$ | $-CH_3$ | |
| 2.48 | Anilino | $C_3H_7(n)$ | $-C_2H_5$ | $-COCH_3$ | |
| 2.49 | Anilino | $C_3H_7(n)$ | $-C_2H_5$ | Benzoyl | |
| 2.50 | Anilino | $C_3H_7(n)$ | $-CH_2CH=CH_2$ | Benzyl | |
| 2.51 | Anilino | $C_3H_7(n)$ | $-CH_2CH=CH_2$ | p-Tolylsulfonyl | |
| 2.52 | Benzylamino | $C_3H_7(n)$ | $-CH_2CH=CH_2$ | $-COCH_3$ | |
| 2.53 | Benzylamino | $C_3H_7(n)$ | $-CH_2CH=CH_2$ | $-CH_3$ | |
| 2.54 | Benzylamino | $C_3H_7(n)$ | $-C_2H_5$ | $-CH_3$ | |
| 2.55 | Benzylamino | $C_3H_7(n)$ | $-C_2H_5$ | $-CH_2CH=CH_2$ | |
| 2.56 | Benzylamino | $C_3H_7(n)$ | $-C_2H_5$ | m-Anisoyl | |
| 2.57 | Benzylamino | $C_3H_7(n)$ | $-C_2H_5$ | Phenylsulfonyl | |
| 2.58 | $N(CH_3)_2$ | $C_3H_7(n)$ | $-C_4H_9(n)$ | Benzoyl | Öl |
| 2.59 | $N(CH_3)_2$ | $C_3H_7(n)$ | $-C_2H_5$ | p-Anisoyl | Smp. 105–108° |
| 2.60 | $N(CH_3)_2$ | $C_3H_7(n)$ | $-C_2H_5$ | Benzyl | Smp. 135–139° |
| 2.61 | $N(CH_3)_2$ | $C_3H_7(n)$ | $-C_2H_5$ | $-CH_2-CH=CH_2$ | Smp. 96–103° |

Beispiel 5:
Formulierungsbeispiele für Wirkstoffe der Formel I (% = Gewichtsprozent)

| a) Spritzpulver | a) | b) | c) |
|---|---|---|---|
| Wirkstoff | 20% | 60% | 0,5% |
| Na-Ligninsulfonat | 5% | 5% | 5% |
| Na-Laurylsulfat | 3% | – | – |
| Na-Diisobutyl-napthalinsulfonat | – | 6% | 6% |
| Octylphenolpoly-äthylenglykoläther (7-8 Mo AeO) | – | 2% | 2% |
| Hochdisperse Kieselsäure | 5% | 27% | 27% |
| Kaolin | 67% | – | – |
| Natriumchlorid | – | – | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff | 10% | 1% |
| Octylphenolpolyäthylenglykoläther (4–5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff | 0,1% | 1% |
| Talkum | 99,9% | – |
| Kaolin | – | 99% |

Man erhält anwendungsfertige Stäubemittel, indem der Wirkstoff mit dem Träger vermischt und auf einer geeigneten Mühle vermahlen wird.

d) Extruder-Granulat

| | a) | b) |
|---|---|---|
| Wirkstoff | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschließend im Luftstrom getrocknet.

e) Umhüllungsgranulat

| | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmäßig aufgetragen. Auf diese Weise erhält man staubfreie Umhüllungs-Granulate.

f) Suspensions-Konzentrat

| | a) | b) |
|---|---|---|
| Wirkstoff | 40% | 5% |
| Äthylenglykol | 10% | 10% |
| Nonylphenolpolyäthylenglykoläther (15 Mol AeO) | 6% | 1% |
| Na-Ligninsulfonat | 10% | 5% |
| Carboxymethylcellulose | 1% | 1% |
| 37%ige wässrige Formaldehyd-Lösung | 0,2% | 0,2% |
| Silikonöl in Form einer 75%igen wässrigen Emulsion | 0,8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5% |
| Isopropylamin | 1% |
| Octylphenolpolyäthylenglykoläther (78 Mol AeO) | 3% |
| Wasser | 91% |

Beispiel 6:
Herbizidwirkung vor dem Auflaufen der Pflanzen

Im Gewächshaus werden Pflanzensamen in Blumentöpfe von 11 cm Durchmesser gesät. Unmittelbar danach wird die Erdoberfläche mit einer wäßrigen Emulsion der Wirkstoffe behandelt. Es wird eine Konzentration von 4 kg Wirkstoffmenge pro Hektar angewendet. Die Töpfe werden dann im Gewächshaus bei einer Temperatur von 22–25 °C und 50–70% relativer Luftfeuchtigkeit gehalten. Nach 3 Wochen wird der Versuch ausgewertet und die Wirkung auf die Versuchspflanzen bewertet. Die Verbindungen der Tabelle I zeigen dabei gute Wirkung, vornehmlich gegen die monocotylen Testpflanzen.

Beispiel 7:
Herbizide Wirkung bei Applikationen der Wirkstoffe nach dem Auflaufen der Pflanzen

Verschiedene Kulturpflanzen und Unkräuter werden aus den Samen in Töpfen im Gewächshaus aufgezogen, bis sie das 4- bis 6-Blatt-Stadium erreicht haben. Dann werden die Pflanzen mit wässerigen Wirkstoffemulsionen (erhalten aus dem 25%igen Emulsionskonzentrat) in einer Dosierung von 4 kg/ha gespritzt. Die behandelten Pflanzen werden dann bei optimalen Bedingungen von Licht, regelmäßigem Begießen, 22–25 °C Temperatur und 50–70%iger relativer Luftfeuchtigkeit gehalten. Die Auswertung des Versuches erfolgt 15 Tage nach der Behandlung. Verbindungen der Tabelle 1 zeigen dabei gute Herbizidwirkung, vornehmlich gegen die monocotylen Testpflanzen.

Beispiel 8:
Wuchshemmung bei tropischen Bodenbedecker-Leguminosen (cover crops)

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wäßrige Emulsion gespritzt. Die Versuchspflanzen werden bei 70% relativer Luftfeuchtigkeit und 6000 lux Kunstlicht, pro Tag 14 Stunden, bei Temperaturen von 27° bei Tag und 21 °C bei Nacht gehalten. 4 Wochen nach der Applikation wird der Versuch ausgewertet. Es werden dabei der Neuzuwachs im Versuch zur Kontrolle abgeschätzt und gewogen und die Phytotoxizität bewertet. In diesem Versuch zeigen die mit den Wirkstoffen der Tabelle 1 behandelten Pflanzen eine deutliche Reduktion des Neuzuwachses (weniger als 20% des Neuzuwachses bei unbehandelten Kontrollpflanzen), ohne daß dabei die Versuchspflanzen geschädigt wurden.

Beispiel 9:
Wuchsregulierung an Sojabohnen

In Kunststoffbehältern mit einem Erde-Torf-Sandgemisch im Verhältnis 6:3:1 werden Sojabohnen der Sorte «Hark» angesät und in eine Klimakammer gegeben. Durch optimale Temperaturwahl, Beleuchtung, Düngerzugabe und Bewässerung entwickeln sich die Pflanzen nach ca. 5 Wochen bis zum 5–6 Trifolia-Blattstadium. Zu diesem Zeitpunkt werden die Pflanzen mit der wäßrigen Brühe eines Wirkstoffs der Formel I bis zur guten Benetzung besprüht. Die Wirkstoffkonzentration beträgt bis zu 100 g AS/ha. Die Auswertung

erfolgt ca. 5 Wochen nach der Applikation des Wirkstoffs. Im Vergleich zu unbehandelten Kontrollpflanzen bewirken die erfindungsgemäßen Wirkstoffe der Formel I eine merkliche Erhöhung der Anzahl und des Gewichts der Schoten im Haupttrieb.

Beispiel 10:
Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprößlinge werden ca. 21 Tage nach der Aussaat mit der wäßrigen Spritzbrühe eines Wirkstoffes der Tabelle 1 besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60–90% der Kontrolle), sowie teilweise eine Zunahme der Stengeldurchmesser auf.

Beispiel 11:
Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wäßrigen Spritzbrühe eines Wirkstoffes der Tabelle 1 besprüht. Die Wirkstoffmenge beträgt umgerechnet bis zu 100 g Aktivsubstanz pro Hektar. 21 Tage nach Applikation wird das Wachstum der Gräser beurteilt.

Die geprüften Verbindungen bewirken eine Reduktion des Neuzuwachses von um 10–30% im Vergleich zur unbehandelten Kontrolle.

**Patentansprüche**

1. Cyclohexenoncarbonsäurederivate der Formel I

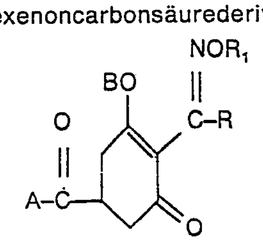

(I),

worin

A einen Rest $-OR_2$ oder $-NR_3R_4$,

R $C_1$–$C_6$ Alkyl oder $C_3$–$C_6$ Cycloalkyl, unsubstituiert oder substituiert durch Halogen, $C_1$–$C_4$ Alkoxy oder $C_1$–$C_4$ Alkylthio,

$R_1$ $C_1$–$C_6$ Alkyl, $C_1$–$C_6$ Halogenalkyl, $C_3$–$C_6$ Alkenyl, $C_3$–$C_6$-Halogenalkenyl oder $C_3$–$C_6$ Alkinyl,

$R_2$ und $R_3$ unabhängig voneinander je Wasserstoff; $C_1$–$C_6$ Alkyl, $C_1$–$C_6$ Halogenalkyl, $C_2$–$C_{10}$ Alkoxyalkyl, $C_2$–$C_{10}$ Alkylthioalkyl; $C_3$–$C_6$ Alkenyl, unsubstituiert oder substituiert durch Halogen, $C_1$–$C_4$ Alkoxy oder $C_1$–$C_4$ Alkylthio; $C_3$–$C_6$ Alkinyl; Phenyl oder $C_1$–$C_6$ Aralkyl, wobei der Phenylkern unsubstituiert oder durch Halogen, $C_1$–$C_4$ Alkyl, $C_1$–$C_4$ Alkoxy, $C_1$–$C_4$ Halogenalkyl, Nitro oder Cyan substituiert ist,

$R_4$ $C_1$–$C_4$ Alkoxy oder dasselbe wie $R_2$

$R_3$ und $R_4$ zusammen mit dem Stickstoffatom, an das sie gebunden sind, bilden auch einen 5–6 gliedrigen Heterocyclus, der im Ring noch ein Sauerstoff- oder Schwefelatom enthalten kann,

B einen Rest $-COR_5$, $-SO_2R_5$ oder $R_6$,

$R_5$ $C_1$–$C_6$Alkyl, $C_3$–$C_6$Alkenyl, $C_3$–$C_6$Alkinyl, sowie Phenyl oder Benzyl welches unsubstituiert oder durch Halogen, $C_1$–$C_4$Alkyl, $C_1$–$C_4$Halogenalkyl, $C_1$–$C_4$Alkoxy, $C_1$–$C_4$Halogenalkoxy, Nitro oder Cyano substituiert sein kann,

$R_6$ $C_1$–$C_6$Alkyl, $C_3$–$C_6$Alkenyl, $C_3$–$C_6$Alkinyl oder auch Benzyl, welches unsubstituiert oder wie oben substituiert sein kann.

2. Cyclohexenoncarbonsäurederivate gemäß Anspruch 1 der Formel Ia

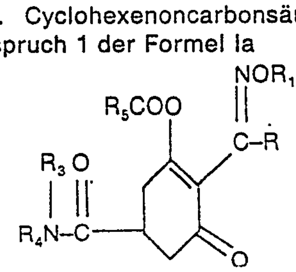

(Ia)

worin R, $R_1$, $R_3$, $R_4$ und $R_5$ die im Anspruch 1 gegebene Bedeutung haben.

3. 3-Acetoxy-4-(1-äthoximino-butyl)-5-oxo-cyclohex-3-en-carbonsäure-dimethylamid gemäß Anspruch 1.

4. 3-Benzyloxy-4-(1-äthoximino-butyl)-5-oxo-cyclohex-3-en-carbonsäure-dimethylamid gemäß Anspruch 1.

5. Cyclohexenoncarbonsäurederivate gemäß Anspruch 1 der Formel Ib

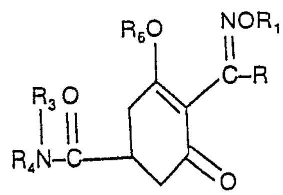

(Ib)

worin R, $R_1$, $R_3$, $R_4$ und $R_6$ die im Anspruch 1 gegebene Bedeutung haben.

6. 4-(1-Äthoximino-butyl)-3-methoxy-5-oxo-cyclohex-3-en-carbonsäure-dimethylamid.

7. Cyclohexenoncarbonsäurederivate gemäß Anspruch 1 der Formel Ic

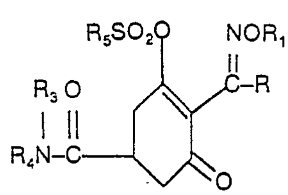

(Ic)

worin R, $R_1$, $R_3$, $R_4$ und $R_5$ die im Anspruch 1 gegebene Bedeutung haben.

8. Cyclohexenoncarbonsäurederivate gemäß Anspruch 1 der Formel Id

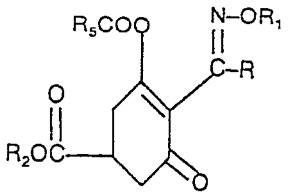

(Id)

worin R, $R_1$, $R_2$ und $R_5$ die im Anspruch 1 gegebene Bedeutung haben.

9. Cyclohexenoncarbonsäurederivate gemäß Anspruch 1 der Formel Ie

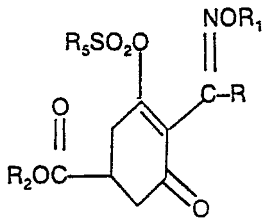

(Ie)

worin R, $R_1$, $R_2$ und $R_5$ die im Anspruch 1 gegebene Bedeutung haben.

10. Cyclohexenoncarbonsäurederivate gemäß Anspruch 1 der Formel If

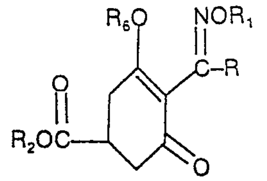

(If)

worin R, $R_1$, $R_2$ und $R_6$ die im Anspruch 1 gegebene Bedeutung haben.

11. Verfahren zur Herstellung der neuen Cyclohexenon-carbonsäurederivate der Formel I, Anspruch 1, dadurch gekennzeichnet, daß man ein 3,5-Cyclohexandioncarbonsäurederivat, respektive ein 3-Hydroxy-5-oxo-cyclohex-3-en-carbonsäurederivat der Formel II

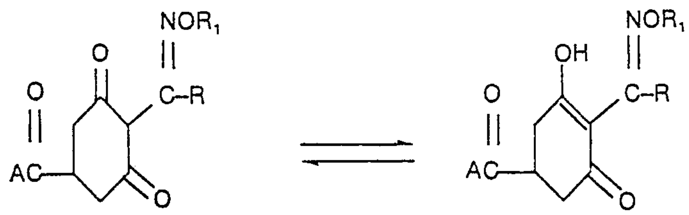

(II)

worin A, R und $R_1$ die im Anspruch 1 gegebene Bedeutung haben, in einem inerten organischen Lösungsmittel, in Gegenwart einer Base, mit einem reaktionsfähigen Ester einer Verbindung der Formel III umsetzt,

BZ (III)

worin B die im Anspruch 1 gegebene Bedeutung hat, und Z eine Abgangsgruppe bedeutet.

12. Verfahren gemäß Anspruch 11, dadurch gekennzeichnet, daß man als reaktionsfähigen Ester der Verbindung der Formel III ein Halogenid oder Acetat verwendet.

13. Ein herbizides und den Pflanzenwuchs regulierendes Mittel, dadurch gekennzeichnet, daß es neben Träger- und/oder anderen Zuschlagstoffen als Wirkstoff mindestens ein Cyclohexenoncarbonsäurederivat der Formel I, Anspruch 1, enthält.

14. Die Verwendung der Cyclohexenon-carbonsäurederivate der Formel I, Anspruch 1, oder sie enthaltender Mittel zur Bekämpfung unerwünschten Pflanzenwachstums.

15. Die Verwendung der Cyclohexenon-carbonsäurederivate der Formel I, Anspruch 1, oder sie enthaltender Mittel zur Regulierung des Pflanzenwachstums.

16. Die Verwendung der Cyclohexenon-carbonsäurederivate der Formel I, Anspruch 1, oder sie enthaltender Mittel zur Wuchshemmung von Gräsern.

17. Verfahren zur selektiven pre- oder postemergenten Bekämpfung von Unkräutern in Nutzpflanzenkulturen, dadurch gekennzeichnet, daß man die Nutzpflanzen oder deren Anbaufläche mit einer wirksamen Menge eines Cyclohexenoncarbonsäurederivates der Formel I, Anspruch 1, oder eines ein solches Derivate enthaltenden Mittels behandelt.

18. Verfahren zur selektiven pre- und postemergenten Bekämpfung von Ungräsern in Nutzpflanzenkulturen, dadurch gekennzeichnet, daß man die Nutzpflanzen oder deren Anbaufläche mit einer wirksamen Menge eines Cyclohexenoncarbonsäurederivates der Formel I, Anspruch 1, oder eines ein solches Derivat enthaltenden Mittels behandelt.

19. Verfahren zur Regulierung des Pflanzenwuchses, dadurch gekennzeichnet, daß man Pflanzen, Pflanzenteile oder Samen mit einer wirksamen Menge eines Cyclohexenon-carbonsäureesters der Formel I, Anspruch 1, oder eines ein solches Derivat enthaltenden Mittels behandelt.

**Claims**

1. Cyclohexenonecarboxylic acid derivatives of formula I

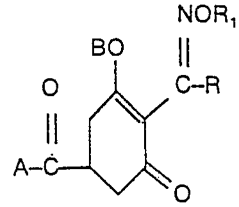

(I),

wherein

A is an $-OR_2$ or $-NR_3R_4$ radical,

R is $C_1$–$C_6$alkyl or $C_3$–$C_6$cycloalkyl each unsubstituted or substituted by halogen, $C_1$–$C_4$alkoxy or $C_1$–$C_4$alkylthio,

$R_1$ is $C_1$–$C_6$alkyl, $C_1$–$C_6$haloalkyl, $C_3$–$C_6$alkenyl, $C_3$–$C_6$haloalkenyl or $C_3$–$C_6$alkynyl,

$R_2$ and $R_3$ are each independently of the other hydrogen; $C_1$–$C_6$alkyl, $C_1$–$C_6$haloalkyl, $C_2$–$C_{10}$alkoxyalkyl, $C_2$–$C_{10}$alkylthioalkyl; or are $C_3$–$C_6$alkenyl which is unsubstituted or substituted by halogen, $C_1$–$C_4$alkoxy or $C_1$–$C_4$alkylthio; or are $C_3$–$C_6$alkynyl, or are phenyl or $C_1$–$C_6$aralkyl, wherein the phenyl nucleus is unsubstituted or substituted by halogen, $C_1$–$C_4$alkyl, $C_1$–$C_4$alkoxy, $C_1$–$C_4$haloalkyl, nitro or cyano,

$R_4$ is $C_1$–$C_4$alkoxy or has the same meaning as $R_2$,

$R_3$ and $R_4$ together with the nitrogen atom to which they are attached also form a 5- or 6-membered heterocycle which may contain an additional oxygen or sulfur atom in the ring,

B is a $-COR_5$, $-SO_2R_5$ or $R_6$ radical,

$R_5$ is $C_1$–$C_6$alkyl, $C_3$–$C_6$alkenyl, $C_3$–$C_6$alkynyl, or also phenyl or benzyl which may be unsubstituted or substituted by halogen, $C_1$–$C_4$ alkyl, $C_1$–$C_4$haloalkyl, $C_1$–$C_4$alkoxy, $C_1$–$C_4$haloalkoxy, nitro or cyano,

$R_6$ is $C_1$–$C_6$alkyl, $C_3$–$C_6$alkenyl, $C_3$–$C_6$alkynyl, or also benzyl which may be unsubstituted or substituted as above.

2. Cyclohexenonecarboxylic acid derivatives according to claim 1 of formula Ia

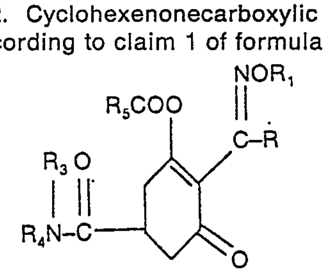

(Ia)

wherein R, $R_1$, $R_3$, $R_4$ and $R_5$ are as defined in claim 1.

3. Dimethyl 3-acetoxy-4-(1-ethoximinobutyl)-5-oxocyclohex-3-enecarboxamide according to claim 1.

4. Dimethyl 3-benzyloxy-4-(1-ethoximinobutyl)-4-oxocyclohex-3-enecarboxamide according to claim 1.

5. Cyclohexenonecarboxylic acid derivatives according to claim 1 of formula Ib

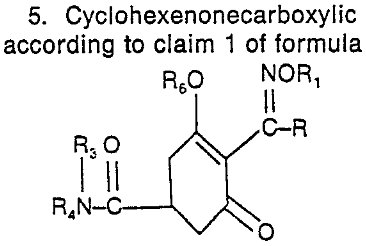

(Ib)

wherein R, $R_1$, $R_3$, $R_4$ and $R_6$ are as defined in claim 1.

6. Dimethyl 4-(1-ethoximinobutyl)-3-methoxy-5-oxocyclohex-3-enecarboxamide.

7. Cyclohexenonecarboxylic acid derivatives according to claim 1 of formula Ic

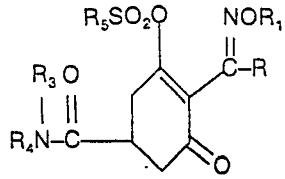

(Ic)

wherein R, $R_1$, $R_3$, $R_4$ and $R_5$ are as defined in claim 1.

8. Cyclohexenonecarboxylic acid derivatives according to claim 1 of formula Id

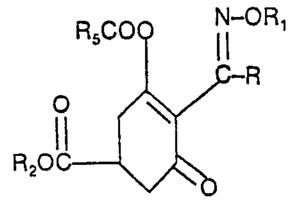

(Id)

wherein R, $R_1$, $R_2$ and $R_5$ are as defined in claim 1.

9. Cyclohexenonecarboxylic acid derivatives according to claim 1 of formula Ie

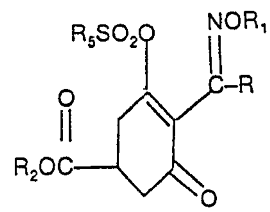

(Ie)

wherein R, $R_1$, $R_2$ and $R_5$ are as defined in claim 1.

10. Cyclohexenonecarboxylic acid derivatives according to claim 1 of formula If

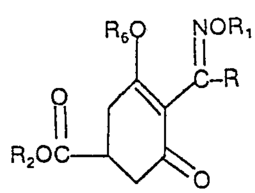

(If)

wherein R, $R_1$, $R_2$ and $R_6$ are as defined in claim 1.

11. A process for the preparation of the novel cyclohexenonecarboxylic acid derivatives of formula I according to claim 1, which process comprises reacting a 3,5-cyclohexanedionecarboxylic acid derivative or a 3-hydroxy-5-oxocyclohex-3-enecarboxylic acid derivative of formula II

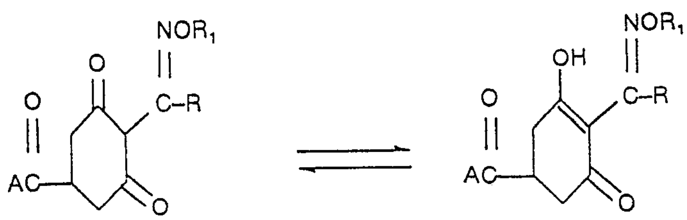

(II)

wherein A, R and $R_1$ are as defined in claim 1, with a reactive ester of a compound of formula III

$$BZ \qquad (III),$$

wherein B is as defined in claim 1 and Z is a leaving group, in an inert organic solvent and in the presence of a base.

12. A process according to claim 11, wherein the reactive ester of the compound of formula III is a halide or an acetate.

13. A herbicidal and plant growth regulating composition which contains, as active ingredient, at least one cyclohexenonecarboxylic acid derivative of formula I according to claim 1, together with carriers and/or other adjuvants.

14. The use of the cyclohexenonecarboxylic acid derivatives of formula I according to claim 1, or of compositions containing them, for controlling undesired plant growth.

15. The use of the cyclohexenonecarboxylic acid derivatives of formula I according to claim 1, or of compositions containing them, for regulating plant growth.

16. The use of the cyclohexenonecarboxylic acid derivatives of formula I according to claim 1, or of compositions containing them, for inhibiting the growth of grasses.

17. A method of selectively controlling weeds pre- or post-emergence in crops of useful plants, which method comprises treating the useful plants or the crop area thereof with an effective amount of a cyclohexenonecarboxylic acid derivative of formula I according to claim 1, or of a composition containing such a derivative.

18. A method of selectively controlling grasses pre- or post-emergence in crops of useful plants, which method comprises treating the useful plants or the crop area thereof with an effective amount of a cyclohexenonecarboxylic acid derivative of formula I according to claim 1, or of a composition containing such a derivative.

19. A method of regulating plant growth, which comprises treating plants, parts of plants or seeds with an effective amount of a cyclohexenonecarboxylic acid ester of formula I according to claim 1, or of a composition containing such a derivative.

**Revendications**

1. Dérivés de l'acide cyclohexénone-carboxylique de formule I

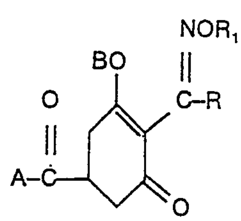

(I),

dans laquelle

A est un reste $-OR_2$ ou $-NR_3R_4$,

R est un alkyle en $C_1$–$C_6$ ou un cycloalkyle en $C_3$–$C_6$, non substitués ou substitués par un ou plusieurs substituants halogènes alcoxy en $C_1$–$C_4$ ou alkylthio en $C_1$–$C_4$,

$R_1$ est un alkyle en $C_1$–$C_6$, un halogénoalkyle en $C_1$–$C_6$, un alkényle en $C_3$–$C_6$, un halogénoalkényle en $C_3$–$C_6$, ou un alkynyle en $C_3$–$C_6$,

$R_2$ et $R_3$, indépendants l'un de l'autre, sont chacun l'hydrogène; un alkyle en $C_1$–$C_6$; un halogènoalkyle en $C_1$–$C_6$; un alcoxy alkyle en $C_2$–$C_{10}$; un alkylthioalkyle en $C_2$–$C_{10}$; un alkényle en $C_3$–$C_6$, non substitués ou substitués par un ou plusieurs substituants halogènes alcoxy en $C_1$–$C_4$ ou alkylthio en $C_1$–$C_4$; un alkinyle en $C_3$–$C_6$; un phényle ou un aralkyle en $C_1$–$C_6$, dans lesquels le noyau phényle est non substitué ou substitué par un ou plusieurs substituants halogènes alkyles en $C_1$–$C_4$, alcoxy en $C_1$–$C_4$, halogénoalkyles en $C_1$–$C_4$, groupes nitro ou cyano,

$R_4$ est un alcoxy en $C_1$–$C_4$, ou est identique à $R_2$,

$R_3$ et $R_4$, avec l'atome d'azote auquel ils sont liés, forment aussi un hétérocycle à 5–6 branches, qui peut comporter dans le cycle encore un atome d'oxygène ou de soufre,

B est un reste $-COR_5$, $-SO_2R_5$ ou $R_6$,

$R_5$ est un alkyle en $C_1$–$C_6$, un alkényle en $C_3$–$C_6$, un alkinyle en $C_3$–$C_6$, ainsi qu'un phényle ou un benzyle, qui peuvent être non substitués, ou substitués par un ou plusieurs substituants halogènes, alkyles en $C_1$–$C_4$, halogénoalkyles en $C_1$–$C_4$, alcoxy en $C_1$–$C_4$, halogénoalcoxy en $C_1$–$C_4$, nitro ou cyano,

$R_6$ est un alkyle en $C_1$–$C_6$, un alkényle en $C_3$–$C_6$, un alkinyle en $C_3$–$C_6$ ou aussi un benzyle qui peuvent être non substitués ou substitués comme précedemment.

2. Dérivés de l'acide cyclohexénone-carboxylique conformes à la revendication 1 de formule Ia

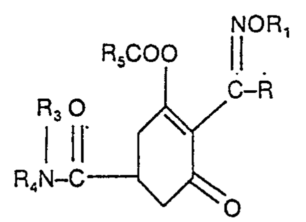

(Ia)

dans laquelle R, $R_1$, $R_3$, $R_4$ et $R_5$ ont la signification donnée dans la revendication 1.

3. Diméthylamide de l'acide
3-acétoxy-4-(1-éthoxyimino-butyl)-5-oxo-cyclohex-3-en-carboxylique
selon la revendication 1.

4. Diméthylamide de l'acide
3-benzyloxy-4-(1-éthoxyimino-butyl)-5-oxo-cyclohex-3-en-carboxylique
selon la revendication 1.

5. Dérivés de l'acide cyclohexénone-carboxylique selon la revendication 1 de formule Ib

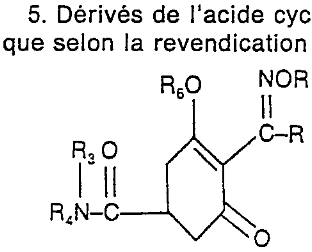

(Ib)

dans laquelle R, $R_1$, $R_3$, $R_4$ et $R_6$ ont la signification donnée dans la revendication 1.

6. Diméthylamide de l'acide 4-(1-éthoxyimino-butyl)-3-méthoxy-5-oxo-cyclohex-3-en-

carboxylique.

7. Dérivés de l'acide cyclohexénone-carboxylique selon la revendication 1 de formule 1c

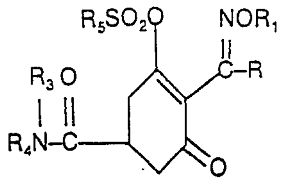

(Ic)

dans laquelle R, $R_1$, $R_3$, $R_4$ et $R_5$ ont la signification donnée dans la revendication 1.

8. Dérivés de l'acide cyclohexénone-carboxylique selon la revendication 1 de formule Id

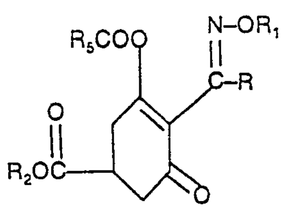

(Id)

dans laquelle R, $R_1$, $R_2$ et $R_5$ ont la signification donnée dans la revendication 1.

9. Dérivés de l'acide cyclohexénone-carboxylique selon la revendication 1 de formule Ie

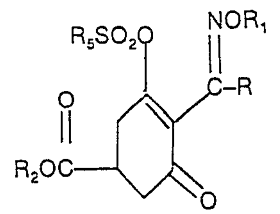

(Ie)

dans laquelle R, $R_1$, $R_2$ et $R_5$ ont la signification donnée dans la revendication 1.

10. Dérivés de l'acide cyclohexénone-carboxylique selon la revendication 1 de formule If

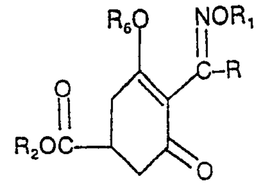

(If)

dans laquelle R, $R_1$, $R_2$ et $R_6$ ont la signification donnée dans la revendication 1.

11. Procédé pour préparer les nouveaux dérivés de l'acide cyclohexénone-carboxylique de formule I, revendication 1, caractérisé en ce que un dérivé de l'acide 3,5-cyclohexanedione-carboxylique, ou un dérivé de l'acide 3-hydroxy-5-oxo-cyclohex-3-en-carboxylique, de formule II

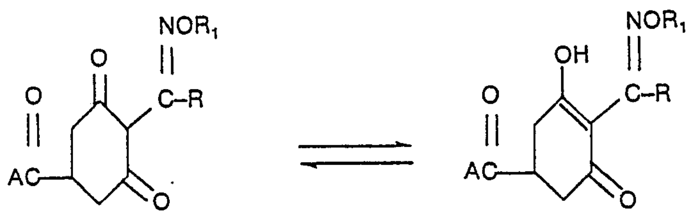

(II)

dans laquelle A, R et $R_1$ ont la signification donnée dans la revendication 1, sont transformés, dans un solvant organique inerte, en présence d'une base, avec un ester réactif d'un composé de formule III,

BZ (III)

dans laquelle B a la signification donnée dans la revendication 1, et Z représente un groupe de départ.

12. Procédé selon la revendication 11, caractérisé en ce que, comme ester réactif du composé de formule III on utilise un halogénure ou un acétate.

13. Herbicide et moyen pour régler la croissance des plantes, caractérisé en ce qu'il comporte comme matière active au moins un dérivé de l'acide cyclohexénone-carboxylique de formule I, revendication 1, en plus d'un support et/ou d'autres additifs.

14. Utilisation des dérivés de l'acide cyclohexénone-carboxylique de formule I, revendication 1, ou moyen les contenant, pour lutter contre la croissance indésirable de plantes.

15. Utilisation des dérivés de l'acide cyclohexénone-carboxylique de formule I, revendication 1, ou moyen les contenant pour régler la croissance des plantes.

16. Utilisation des dérivés de l'acide cyclohexénone-carboxylique de formule I, revendication 1, ou moyen les contenant, pour limiter la croissance des graminées.

17. Procédé pour lutter de façon sélective contre les mauvaises herbes, avant, ou après leur apparition, dans les cultures de plantes utiles, caractérisé en ce que les plantes utiles ou leur surface de culture sont traitées avec une quantité active d'un dérivé de l'acide cyclohexénone-carboxylique de formule I, revendication 1, ou d'un moyen contenant un tel dérivé.

18. Procédé pour lutter de façon sélective contre les mauvaises herbes, avant et après leur apparition, dans les cultures de plantes utiles, caractérisé en ce que l'on traite les plantes utiles ou leur surface de culture avec une quantité active d'un dérivé de l'acide cyclohexénone-carboxylique de formule I, revendication 1, ou d'un moyen contenant un tel dérivé.

19. Procédé pour regler la croissance des plantes, caractérisé en ce que l'on traite des plantes, des plants ou des graînes, avec une quantité active d'un ester de l'acide cyclohexénone-carboxylique de formule I, revendication 1, ou d'un moyen contenant un tel dérivé.